(19) Europäisches Patentamt
European Patent Office
Office européen des brevets

(11) EP 1 620 718 B1

(12) **EUROPEAN PATENT SPECIFICATION**

(45) Date of publication and mention of the grant of the patent:
**29.10.2008 Bulletin 2008/44**

(21) Application number: **04731652.6**

(22) Date of filing: **07.05.2004**

(51) Int Cl.:
***G01N 31/22*** *(2006.01)* ***G01N 21/78*** *(2006.01)*
***C11D 1/00*** *(2006.01)* ***C07K 1/30*** *(2006.01)*

(86) International application number:
**PCT/GB2004/001992**

(87) International publication number:
**WO 2004/099234 (18.11.2004 Gazette 2004/47)**

# (54) METHOD OF QUANTIFYING THE SURFACTANT OF A PROTEIN PREPARATION

VERFAHREN ZUR TENSID-QUANTIFIZIERUNG EINES PROTEINPRÄPARATES

PROCEDE DE QUANTIFICATION DE TENSOACTIF D'UNE PREPARATION PROTEINIQUE

(84) Designated Contracting States:
**AT BE BG CH CY CZ DE DK EE ES FI FR GB GR HU IE IT LI LU MC NL PL PT RO SE SI SK TR**

(30) Priority: **07.05.2003 GB 0310347**

(43) Date of publication of application:
**01.02.2006 Bulletin 2006/05**

(73) Proprietor: **Novozymes Biopharma UK Limited Nottingham NG7 1FD (GB)**

(72) Inventors:
- **MORTON, Philip Harvey, Novozymes Biopharma UK Limited Nottingham NG7 1FD (GB)**
- **CAMERON, Jason, Novozymes Biopharma UK Limited Nottingham NG7 1FD (GB)**

(56) References cited:
**WO-A-02/086492** **DE-A- 2 435 759**

- **Abstract P-21-T by Lanteigne, D. & Kobayashi, K. WCBP 2002, 6th Symposium on the interface of regulatory and analytical sciences for biotechnology health products (January 27-30, 2002) URL: http://www.casss.org/meetings/ 2002WCBP/cal lforabstracts.htm [retrieve 16.11.04] XP002306952 cited in the application**
- **GAREWAL H S: "A PROCEDURE FOR THE ESTIMATION OF MICROGRAM QUANTITIES OF TRITON X-100" ANALYTICAL BIOCHEMISTRY, vol. 54, no. 2, 1973, pages 319-324, XP009040085 ISSN: 0003-2697 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 620 718 B1

## Description

The present invention relates to a method for removing components from a surfactant preparation, and for assaying for a surfactant in a preparation.

Particle formation following heat treatment of protein preparations, such as human serum albumin (HSA) and recombinant human albumin (rHA), is a known problem (EP 0 341 103). Particles are thought to form through protein denaturation at the air/liquid interface and at other hydrophobic surfaces (Manning, M.C., Patel, K. & Borchart, R.T. (1989), Pharmaceutical Research, 6, 903-918; Thurow, H. & Geisen, K. (1984), Diabetologia, 27, 212-218). Particle formation can be inhibited by the addition of surfactants to the protein preparation.

It is possible to use polysorbate 80 at a concentration of 10-20 $\mu g.mL^{-1}$ or more as a formulant for rHA final product to prevent particle formation. EP 0 341 103 discusses the use of various surfactants at concentrations of up to 50 $mg.L^{-1}$ for stabilising human albumin solutions. Many other pharmaceutical protein preparations include surfactants. For example, Orthoclone™ OKT3 (Janssen-Cilag GmbH, Germany) contains polysorbate 80 at about $0.2mg.mL^{-1}$; Activase™ 50 (Genentech, Inc., CA., USA) contains polysorbate 80 at ≤4mg per vial in a total volume of 50mL; Vepesid™ J 100 (Bristol Laboratories NJ, USA) contains amongst other ingredients 400mg polysorbate 80; and NovoSeven™ 240 (Novo Nordisk A/S, Denmark) contains amongst other ingredients 0.65mg polysorbate 80.

Thus, surfactants can represent a significant formulant in a protein preparation including pharmaceutical protein preparations. As such, there is a regulatory requirement to assay for them in the final product. The accuracy of the assay is particularly important in the case of pharmaceutical preparations. However, it is not possible to assess surfactant content accurately in the presence of protein because the detection techniques employed for surfactants, such as spectroscopy, high performance liquid chromatography, interfacial tensiometry, capillary electrophoresis, total organic carbon (TOC) titrations, and TLC etc., also detect protein. Thus the protein content leads to an over-estimation of surfactant content.

Garewal (Anal. Biochem., 54, 319-324, 1973) provided a method for assaying for the surfactant content of an aqueous protein solution. The protocol teaches, as a first step, the addition of ethanol to disrupt micelles, followed by the addition of ammonium cobaltothiocyanate (ACT). The method exemplified by Garewal used an aqueous solution of the Triton X-100 surfactant, to which ACT binds and forms a blue-coloured complex. Garewal then added a non-miscible organic phase (ethylene dichloride) in which the ACT-Triton X-100 complex is soluble. The complex migrates into the organic phase and the organic phase is separated from the aqueous phase. Finally, the Triton X-100 content of the organic phase is determined by recording the spectrum of the organic phase from 580nm to 700nm; the difference in absorbance at 622 nm and 687 nm is said to be proportional to the amount of Triton surfactant present.

Garewal investigated the effect on the efficacy of the method of introducing a protein, bovine serum albumin (BSA), into the aqueous solution of Triton X-100. BSA concentrations up to 666 $\mu g.mL^{-1}$ were investigated. Use of lower BSA concentrations, e.g. $267\mu g.mL^{-1}$, caused a reduction in the extraction efficacy to about 85%, but increasing protein concentration up to $666\mu g.mL^{-1}$ was found not to cause any further significant reduction in extraction efficacy. Garewal concluded that, since poly(ethylene oxide) groups, with which ACT reacts, are rare in biological components (e.g. proteins), a minimum of interference is expected and the method described therein is appropriate for biochemical assays.

The method of Garewal has remained the method of choice for surfactant quantitation in biological preparations for the last 30 years. With minor modifications, the method of Garewal was presented at WCBP 2002, 6th Symposium on the Interface of Regulatory and Analytical Sciences for Biotechnology Health Products (January 27-30, 2002) by Lanteigne, D. & Kobayashi, K. of Biogen, Inc., Cambridge, MA USA in a poster entitled "Quantitative Determination of Polysorbate in Formulated Protein-Based Biopharmaceuticals by a Direct Colorimetric Method". The poster describes an assay for polysorbate 80 (sold under the trademark "Tween 80") in a monoclonal antibody preparation at 52 $mg.mL^{-1}$. Lanteigne & Kobayashi state that, where samples contain a 'high' concentration of protein (e.g. 52 $mg.mL^{-1}$), then it is necessary to use "a protein removal step to eliminate possible interference by the active drug substance" (i.e. by the protein). Lanteigne & Kobayashi address this by ethanol precipitation of the protein in a preparation, involving overnight incubation of the sample at minus 30 °C (plus centrifugation and isolation of the supernatant), prior to complexing the surfactant by the addition of ACT and extraction of the ACT-surfactant complex using dichloromethane as an organic liquid phase.

However, following investigations, we surprisingly found that, contrary to their teachings, the Lanteigne & Kobayashi method does not provide an accurate assay for the surfactant content of protein solutions. The accuracy of this method, and also of the basic Garewal method, is particularly poor at a higher protein concentration. For example, as described below (see Comparative Example 1), the method of Garewal gave misleading results when a sample was tested in which the protein content of a surfactant solution was greater than 50 $mg.mL^{-1}$. The method of Garewal is not expected to provide an accurate surfactant assay with solutions containing protein at 200 $mg.mL^{-1}$. This is because these methods fail to remove protein components from the sample of surfactant at the point of analysis. Moreover, we have demonstrated that the ethanol addition steps proposed by Garewal and by Lanteigne & Kobayashi result in unacceptably high losses of surfactant, and so provide unreliable data. We have also demonstrated that removing the protein

content of a surfactant preparation leads to unacceptable surfactant losses when extracting the surfactant using the ACT/dichloromethane process described by Lanteigne & Kobayashi.

To overcome this unexpected problem, we have devised a new method for separating protein and other components from a surfactant in a given sample, thereby to provide a more complete surfactant preparation which, when analysed, provides a result that is more representative of the actual surfactant content of the original sample from which it was taken. Moreover, the method of the present invention does not require the time-consuming step of overnight incubation of a sample in order to remove protein and so is a more efficient method to perform than that described by Lanteigne & Kobayashi.

Accordingly, in a first aspect of the present invention, there is provided a method for removing proteinaceous components from a liquid-phase surfactant preparation comprising -

(a) providing a liquid-phase surfactant preparation containing a proteinaceous component;

(b) adding a complexing agent to the preparation of step (a) and allowing the complexing agent to form a complex with the surfactant;

(c) simultaneously with step (b), or subsequently, adding a miscible precipitating agent to the preparation of step (a) or the product of step (b), respectively, to form a liquid-phase reaction mixture and allowing the miscible precipitating agent to precipitate the proteinaceous component within the liquid-phase reaction mixture; and

(d) separating the said complex from the precipitated proteinaceous component in the product of step (c) to provide a purified liquid-phase surfactant preparation;

wherein the complex remains in solution within the liquid-phase reaction mixture, and wherein step (d) retains the complex in the liquid phase.

Any surfactant type can be purified by a method according to the first aspect of the present invention. A surfactant is a molecule that can act to reduce the surface tension of a liquid. Surface tension is the force acting on the surface of a liquid, tending to minimise the area of the surface; quantitatively, it is the force that appears to act across a line of unit length on the surface. The surface tension of water is 72 dyne/cm when measured at room temperature (20°) using a tensiometer; a surfactant can reduce this value, typically to a surface tension of no more than 50 dyne/cm, for example about 30-50 dyne/cm.

Typically, the surfactant will be non-ionic, i.e. having an uncharged hydrophilic head group. Examples of non-ionic surfactants include surfactants having a poly(alkylene oxide) group, such as a poly(ethylene oxide) group, an alcohol group or another polar group. Suitable non-ionic surfactants may have a hydrophobic group and a reactive hydrogen atom, for example aliphatic alcohols, acids, amides or alkyl phenols with alkylene oxides, especially ethylene oxide either alone or with propylene oxide.

Thus the non-ionic surfactant may be a condensate between an alkylphenol and an alkylene oxide; a polyoxyalkylene sorbitan oleate; or a polyoxyalkylene glycol.

Specific non-ionic surfactant compounds include alkyl ($C_6$-$C_{22}$) phenols-ethylene oxide condensates, the condensation products of aliphatic ($C_8$-$C_{18}$) primary or secondary linear or branched alcohols with ethylene oxide, and products made by condensation of ethylene oxide with the reaction products of propylene oxide and ethylenediamine. Other non-ionic surfactant compounds include long-chain tertiary amine oxides, long-chain tertiary phosphine oxides and dialkyl sulphoxides. A non-ionic surfactant may also be a sugar amide, such as a polysaccharide amide, such as one of the lactobionamides described in US 5,389,279 or one of the sugar amides described in US 5,009,814. Other typical surfactants of this type include Igepal DM 730, Igepal DM 530, Igepal DM 210, Igepal CO 880, Igepal CO 530, polyoxyethyleneglycols, including compounds sold under the Trade Mark Brij (such as polyoxyethylene (4) lauryl ether (Brij 30), lauryl ether (Brij 35), polyoxyethylene (20) cetyl ether (Brij 58), polyoxyethylene (20) stearyl ether (Brij 78) and polyoxyethylene (20) oleyl ether (Brij 92)), and polyoxyethylene fatty acid esters, including compounds sold under the Trade Mark Myrj (such as Myrj 51). Typical non-ionic surfactants include polyoxyethylene octyl phenol (such as Triton X-100); alkylphenoxypolyethoxy (3) ethanol, polyoxyethylene (20) sorbitan monolaurate (Tween 20), polyoxyethylene (20) sorbitan monopalmitate (Tween 40), polyoxyethylene (20) sorbitan monostearate (Tween 60), polyoxyethylene (20) sorbitan tristearate (Tween 65), polyoxyethylene (20) sorbitan monooleate (Tween 80), polyoxyethylene (20) sorbitan trioleate (Tween 85), polyoxyethylene (20) palmitate (G2079), polyoxyethylene (20) lauryl ether; polyoxyethylene (23), polyoxyethylene (25) hydrogenated castor oil (G1292) and polyoxyethylene (25) oxypropylene monostearate (G2162).

Other surfactants suitable for use in a method according to the first aspect of the invention may be:

- anionic, with negatively charged head groups. Examples of anionic surfactants include long-chain fatty acids, sulphosuccinates, alkyl sulphates, phosphates and sulphonates, such as sodium dodecyl sulphate, sodium cholate,

sodium deoxycholate, and sodium taurocholate.

- cationic, with positively charged head groups. Examples of cationic surfactants include protonated long-chain amines and long-chain quaternary ammonium compounds, such as hexadecyltrimethyl ammonium bromide (Cetavlon), cetyltrimethyl ammonium bromide, and N-hexadecylpyridinium chloride.

- amphoteric, with zwitterionic head groups. Examples of amphoteric surfactants include betaines and certain lecithins.

The surfactants may have one or more alkylene oxide groups. Any alkylene oxide group may be present, such as ethylene oxide, propylene oxide, butylene oxide and the like. Ethylene oxide groups are common in commercially available surfactants. Multiple alkylene oxide groups may be present as a polymer (e.g. a homopolymer, co-polymer or block co-polymer), i.e. as a poly(alkylene oxide) group, such as the homopolymeric poly(ethylene oxide) group. It is common for a surfactant to contain six or more alkylene oxide groups, although it is possible for this method to work with surfactants having fewer, such as 5, 4, 3, 2 or 1 alkylene oxide group(s). The surfactant may be a non-ionic surfactant having one or more poly(ethylene oxide) groups, such as polysorbates, octylphenol ethylene oxide condensate, ethylene oxide/polypropylene oxide block copolymers, polyoxyalkylene glycols, polyoxyethylene hardened castor oil, polyoxyethylene glycerol fatty acid esters, polyoxyethylene alkyl ethers, polyoxyethylene polyoxypropylene glycol, polyoxyethylene alkyl allyl ethers and the like.

Polysorbates (also known as polyoxyethylene sorbitan esters, as sold under the Registered Trade Mark Tween) are non-ionic surfactants derived from sorbitan esters (Becher, P. "Polyol Surfactants" in Nonionic Surfactants, Schick, M.J. Ed. (Dekker, New York, 1967), page 247-299; Chislett, L.R. & Walford, J. (1976) Int Flavours Food Addit., 7, 61; Varma, R.K. et al (1985) Arzneimittel-Forsch, 35, 804). Preferred polysorbates include polysorbate 20, 21, 40, 60, 65, 80, 81, 85 and the like. A particularly preferred surfactant is polysorbate 80, which has the general formula (I) -

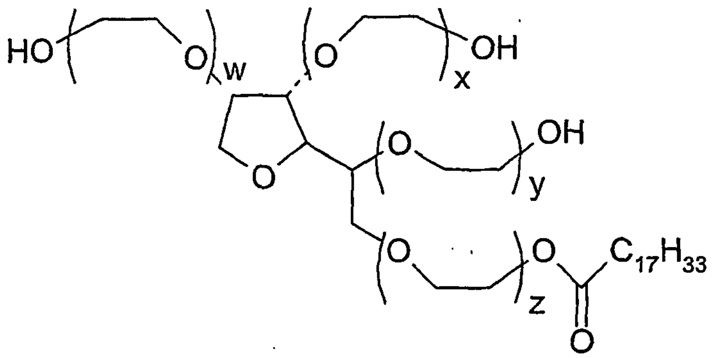

Sum of w, x, y and z is 20

(I).

Octylphenoxy polyethoxyethanol (also known as octoxynol, and sold under the Trade Marks of Triton X, Igepal CA and Polytergent G) is a non-ionic surfactant that may be prepared by reacting isooctylphenol with an alkylene oxide, such as ethylene oxide. The average number of ethylene oxide units (n) per molecule of common commercially available octoxynol typically varies between 5 and 15. The general formula is represented by formula (II) below -

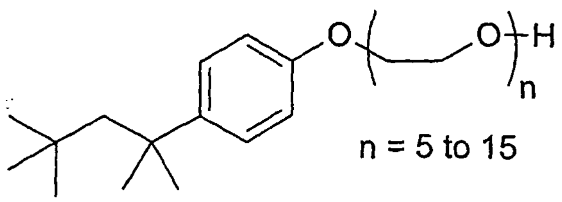

n = 5 to 15

(II).

In a typical such surfactant, sold as Triton X-100, n is about 9.5.

Polyethylene polypropylene glycols (also known as poloxamers and sold under the registered trade mark Pluronic) are a series of nonionic surfactants with the general formula represented by formula (III) below -

$$HO(CH_2CH_2O)_a(CH\text{-}(CH_3)CH_2O)_b(CH_2CH_2O)_cH \qquad (III)$$

where b is at least 15 and $(CH_2CH_2O)_a$+ $(CH_2CH_2O)_c$ is varied from 20 to 90% by weight. The molecular weight ranges from 1,000 to 16,000 $g.mol^{-1}$ or more. For a review of poloxamers, see Schmolka, I.R. (1967) *Am. Perfumer Cosmet.*, **82**(7), 25-30. Examples of particular poloxamers include "Pluronic L62LF", wherein a = 7, b = 30, c = 7; "Pluronic F68" wherein a = 75, b = 30, c = 75; and "Pluronic L101" wherein a = 7, b = 54, c=7.

Surfactants for use with a method according to the first aspect of the present invention may additionally contain one or more linear or branched hydrocarbon chains. Hydrocarbon chains typically found in commercially available surfactants include fatty acids. A fatty acid usually has at least six carbon atoms in the hydrocarbon backbone, and larger backbones are common, such as $C_{16}$ and $C_{18}$. Accordingly, the hydrocarbon chain may be an oleic acid (i.e. $C_{16}$ fatty acid) group. The surfactant may contain both a poly(alkylene oxide) group and a hydrocarbon chain. For example, polysorbates contain both poly(ethylene oxide) groups and an oleic acid group; octoxynol comprises a branched hydrocarbon chain and poly(ethylene oxide) groups.

The proteinaceous component may comprise any proteinaceous molecule that is undesired in any purified surfactant preparation that is prepared from the starting material. In particular the component may be one that interferes with the accuracy of any subsequent surfactant quantification. A component is proteinaceous if it comprises or consists of a peptide, polypeptide or protein. The phrase "peptide, polypeptide or protein" includes any polymer of amino acids, whether naturally occurring or artificial, preferably joined by peptide bonds. Preferably a peptide, polypeptide or protein will be at least 10, 20, 30, 40, 50, 60, 70, 80, 90 or 100 amino acids in length. The proteinaceous component may be a naturally occurring or recombinantly produced protein, such as albumin, an albumin fusion protein such as mentioned in WO 01/77137, a monoclonal antibody, etoposide, a serum protein (such as a blood clotting factor), antistasin, tick anticoagulant peptide or any one or more of the albumin "fusion partners" disclosed in WO 01/77137, as an individual protein separate from albumin.

Unlike prior art methods, the method of the first aspect of the invention is capable of efficiently separating surfactant from a highly concentrated proteinaceous component, which for example, may be present in the liquid-phase surfactant preparation of step (a) at a concentration of at least 50, 75, 100, 150, 200 mg/ml, where component levels are measured in weight per volume of surfactant preparation.

It may be appropriate to measure the ratio of surfactant to proteinaceous component in the liquid-phase surfactant preparation of step (a). Accordingly, the ratio of surfactant to proteinaceous component, when expressed as mass of surfactant molecules per mass of proteinaceous component molecules (i.e. ppm) present in the liquid-phase surfactant preparation of step (a) may be less than 4,800 ppm, such as less than 4,500 ppm, 4,000 ppm, 3,500 ppm, 3,000 ppm, 2,500 ppm, 2,000 ppm, 1,500 ppm, 1,000 ppm, 900 ppm, 800 ppm, 700 ppm, 600 ppm, 500 ppm, 400 ppm, 300 ppm, 200 ppm, 110 ppm, 100 ppm, 90 ppm, 80 ppm, 75 ppm, 70 ppm, 60 ppm, 50 ppm, 40 ppm, 30 ppm, 20 ppm, 18 ppm, 17 ppm, 16 ppm, 15 ppm, 14 ppm, 13 ppm, 12 ppm, 11 ppm, 10 ppm, 9 ppm, 8 ppm, 7 ppm, 6 ppm, 5 ppm or less.

The term "liquid-phase surfactant preparation" includes any liquid-phase preparation comprising a surfactant. The preparation may be aqueous.

By "providing", in the context of providing a liquid-phase surfactant preparation, we include taking a whole sample, an aliquot from a larger preparation, or one of batch of samples prepared from the same basic lot.

The term "complexing agent" includes any compound capable of modifying the hydrophobic characteristic of a surfactant through its ability to form weak bonds with on or more surfactant molecules. A surfactant as defined above under the conditions of the process. Typically the complexing agent will be a compound that contains a polyvalent metal ion, such as a transition metal ion. For example, the metal ion may be a group VI, VII, VIII, IX or X transition metal ion, such as yttrium, zirconium, niobium, molybdenum, technetium, ruthenium, rhodium, palladium, silver, cadmium, hafnium, tantalum, tungsten, rhenium, osmium, iridium, platinum, gold, mercury, although preferred transition metal ions are 3d transition metal ions such as cobalt, iron, copper, zinc, nickel, manganese, chromium, vanadium, titanium and scandium. Cobalt compounds may be used as the complexing agent. Accordingly, the complexing agent may be ammonium cobaltothiocyanate (ACT). ACT is an appropriate complexing agent to use in order to complex a surfactant having an alkylene oxide or poly(alkylene oxide) (e.g. ethylene oxide or poly(ethylene oxide)) group.

Similarly, iron compounds, such as iron (III) thiocyanate, may be used as the complexing agent.

Unlike prior art methods, the method of the present invention does not rely on the formation of a colour complex to assess surfactant presence. Therefore, the complexing agents used in the present invention do not necessarily need to form coloured complexes.

An effective amount of the complexing agent is added to the liquid-phase surfactant preparation. In other words, the amount of complexing agent added is sufficient to complex substantially all of the surfactant in the liquid-phase surfactant preparation. Typically it is added in excess. The amount of complexing agent required to complex substantially all of the surfactant in the liquid-phase surfactant preparation can be determined by empirical testing of the complexing agent with an uncontaminated solution of the surfactant.

By "allowing the complexing agent to form a complex with the surfactant" we mean that at least some of the

complexing agent complexes with at least some of the surfactant. Typically, after the complexing agent is added to the liquid-phase surfactant preparation, the preparation is mixed to disperse the complexing agent within the preparation. The optimum conditions to allow complexing to occur will depend on the nature of the surfactant and the nature of the complexing agent, and will typically include modification of temperature, pressure, pH and/or ionic strength of the liquid phase. Useful conditions for complexing may include neutral pH and low ionic strength (Crabb & Persinger, 1961, Journal of the American Oil Chemist's Society, 41, 752-755). For example, in the case of the surfactant being polysorbate 80 and the complexing agent being ACT, suitable conditions for allowing the complexing agent to form a complex with the surfactant are as set out below in the examples.

Following formation, the complex remains in solution within the liquid-phase. Hence under the conditions used for allowing the complexing agent to form a complex with the surfactant (but in the absence of any protein), substantially none of the complex forms a precipitate. A complex can be said to remain in solution if the amount of surfactant that can be collected in the pellet as a precipitate, by centrifugation of the liquid phase at 47,800 g for 15 minutes at 4°C, is less than 20%, 15%, 10%, 5%, 2%, 1%, 0.5% or 0.1% by weight of the surfactant collected in the supernatant after centrifugation when determined using HPLC as described in the examples below. Lower percentage values are preferred.

A "precipitating agent" is any agent that causes a component other than the surfactant to precipitate. The precipitating agent must be "miscible" within the liquid-phase surfactant preparation in order to perform its function. In other words, under the conditions used, the precipitating agent must not form a separate liquid or solid phase that is immiscible with the liquid-phase surfactant preparation. Preferably the precipitating agent is miscible in an aqueous liquid-phase surfactant preparation. To be water-miscible, a precipitating agent will commonly have a polar region. Typically the precipitating agent is an organic water-miscible solvent. Examples of water-miscible precipitating agents include polar protic solvents and polar aprotic solvents such as alcohols, cyanoalkyls, amines, amides, carboxylic acids, aldehydes, ketones, glycols, ethers, alkylhalides and aromatic hydrocarbons. Preferred precipitating agents include acetone, acetonitrile, isopropanol, methanol and ethanol. Acetonitrile provides a good balance between surfactant yield and contaminant carry-over. Moreover, acetonitrile has advantages over acetone including -

(a) the use of acetone requires the use of glassware for supernatant manipulation which could be contaminated with detergent from cleaning, whereas the use of acetonitrile allows disposable plastic containers to be used, thereby minimising the risk of contamination; and

(b) acetone also has a flash point of -18°C, lower than typical centrifugation temperatures, and for safety it is better to use acetonitrile, which has a flash point of +13°C.

The precipitating agent is added simultaneously or, more generally, after, but not before, the complexing agent is added to the liquid-phase surfactant preparation. This is an important difference between the present invention and the prior art. Both Garewal (*op. cit.*) and Lanteigne & Kobayashi (*op. cit.*) added a precipitating agent (ethanol) to the surfactant preparation before the complexing agent (ACT) is added. This causes some surfactant to be lost from solution, as it is carried into the precipitate. Hence, the resulting quantification of the surfactant in the supernatant is an inaccurate measure of the amount of surfactant in the starting preparation. Without being bound by theory, it is believed that by adding a precipitating agent simultaneously or, more generally, after, but not before, the complexing agent, the efficacy of the proteinaceous component removal is improved. The complexing agent keeps substantially all of the surfactant in solution whilst the proteinaceous component is precipitated.

In some cases, the subsequent addition of the precipitating agent may enhance the effect of the complexing agent and result in a greater degree of complex formation between the surfactant and the complexing agent. Without being bound by theory, we believe that this is because the precipitating agent further separates the surfactant from the proteinaceous component, thereby allowing improved complexing of the surfactant by the complexing agent.

When "allowing the miscible precipitating agent to precipitate the proteinaceous component within the liquid-phase reaction mixture", the liquid-phase reaction mixture may be incubated under conditions that favour the precipitation of the proteinaceous component but do not substantially disturb the complex. The actual conditions used will depend on the identity of the particular components within the system in question. The person skilled in the art is capable of determining appropriate conditions for any given combination of system components by empirical testing.

The complex remains in solution within the liquid-phase reaction mixture. In this context, the complex "remains in solution" if the amount of surfactant that can be collected in the pellet as a precipitate, by centrifugation of the liquid-phase reaction mixture at 47,800 g for 15 minutes at 4°C, is less than 20%, 15%, 10%, 5%, 2%, 1%, 0.5% or 0.1% by weight of the surfactant collected in the supernatant after centrifugation when determined using HPLC as described in the examples below. Lower percentage values are preferred.

The step of "separating the said complex from the precipitated proteinaceous component in the product of step (c)" can be effected by any suitable method known in the art for separating precipitate from a solution, so long as it "retains the complex in the liquid phase". Substantially all of the complex is retained in the liquid-phase product of step

(c). For the avoidance of doubt, the complex is not retained in the liquid phase if it is partitioned into a separate non-miscible liquid phase. This is another important difference between the method of the present invention and the methods of Garewal (*op. cit.*) and Lanteigne & Kobayashi (*op. cit.*). The methods of Garewal (*op. cit.*) and Lanteigne & Kobayashi (*op. cit.*) isolate the complexed surfactant from an aqueous solution by the addition of an immiscible organic phase (either ethylene dichloride or dichloromethane). Without being bound by theory, we believe that the partition of surfactant complex into a separate liquid phase results in a large contaminant carry-over. By contrast, we do not rely on this form of complex isolation and, consequently, achieve a greater removal of proteinaceous components.

The separating step is typically performed by centrifuging the reaction mixture, such that the precipitated proteinaceous component forms a pellet and the complex is retained in the supernatant, and separating the supernatant from the pellet. Optimal centrifugation parameters such as g and duration will vary depending on the nature of the precipitate formed. Guidance can be taken from the examples below, although the person skilled in the art is capable of determining appropriate conditions by empirical testing.

However, the person skilled in the art will be aware that numerous other methods are available in the art to separate a liquid phase preparation from a precipitate, such as filtration.

The product of the separation step is a purified liquid-phase surfactant preparation. By "purified liquid-phase surfactant preparation" is included the meaning of a liquid-phase surfactant preparation that is substantially free of precipitated proteinaceous component. In this context, a liquid-phase surfactant preparation is substantially free of precipitated proteinaceous component if it can be applied to a hydrophobic solid phase extraction cartridge under conditions defined in the examples below without blocking the cartridge or significantly affecting the purity of the surfactant after SPE purification.

A method according to the first aspect of the present invention may comprise one or more additional purification steps to further purify the surfactant in the purified liquid-phase surfactant preparation. Any suitable methods may be used.

In one embodiment, the method according to the first aspect of the present invention comprises the additional step of non-covalently binding the complex in the purified liquid-phase surfactant preparation to a solid phase. Typically a hydrophobic solid phase is used, as this adsorbs the surfactant. Alternatively, a hydrophilic solid phase may be used, which adsorbs remaining proteinaceous component in the purified liquid-phase surfactant preparation without retaining the surfactant, thereby allowing the surfactant to be collected as an eluate.

It may be helpful if the complex is dissociated prior to exposure to the solid phase. The skilled person is well aware of methods to dissociate the complex. The particular details depend on the nature of the surfactant and complexing agent. For example, a chelating agent may be used. Typically the chelating agent will compete with the surfactant to bind to the polyvalent metal ion of the complexing agent. Accordingly, where the complexing agent is ACT (i.e. the polyvalent metal ion is cobalt), a suitable method of dissociating the complex is by the addition of a chelating agent such as ethylenediamine tetra-acetic acid (EDTA) to the purified liquid-phase surfactant.

In one embodiment, the solid phase used in the additional step is a solid phase extraction (SPE) cartridge or disk.

The SPE cartridge or disk may be hydrophobic. Examples of hydrophobic SPE cartridges and disks include a polystyrene divinylbenzene (e.g. the Bakerbond SDB1 columns exemplified below, the Licrolut EN PDBV cartridges supplied by Merck, or StrattaX supplied by Phenomenex) or a $C_{2-24}$ alkyl cartridge.

In a method according to the first aspect of the invention, where the surfactant is non-covalently bound to a solid matrix, the solid matrix may be washed with a liquid that allows the bound surfactant to remain bound to the matrix whilst any remaining proteinaceous component is washed away. Suitable wash liquids are well known in the art and are commercially available. Suitable wash liquids include isopropanol, hexane and acetonitrile. It may be helpful for a wash to be acidic or alkaline. For example, acetic acid can be presented in hexane at an appropriate concentration, such as 0.1 % (v/v), to provide an acidic wash. Ammonium, or triethylamine, can be presented in hexane at an appropriate concentration, such as 0.5% (v/v) ammonium or 1% (v/v) triethylamine, to provide an alkaline wash. The appropriate wash conditions can be determined by the skilled person dependent on the nature of the surfactant and the solid phase.

The matrix may be washed with a liquid that does not remove the surfactant from the matrix. Typically, an appropriate wash liquid may be sufficiently hydrophilic as to not disrupt the interaction of the surfactant with the matrix or alternatively may be sufficiently hydrophobic as to precipitate the surfactant in solution. A method of determining a suitable wash liquid can be performed as follows. A liquid is considered to precipitate a surfactant if at least 90%, 92%, 94%, 96%, 98%, 99% or substantially 100% of the surfactant (e.g. polysorbate 80) can be recovered from the matrix (e.g. a polystyrene divinylbenzene SPE cartridge, such as the Bakerbond SDB1 columns exemplified below, the Licrolut EN PDBV cartridges supplied by Merck, or StrattaX supplied by Phenomenex, or equivalents thereof) under the following conditions -

(a) The cartridge is prepared according to step 2(v)(a) of Example 2 below.

(b) The cartridge is loaded (at approximately 0.5 mL. $min^{-1}$) with 10mL of a 15 $\mu g.mL^{-1}$ aqueous surfactant solution.

(c) The cartridge is washed with 3 x 1 mL of the wash liquid in question. The cartridge is fully dried by passing air through it under vacuum for at least 30 seconds.

(d) Surfactant on each cartridge is eluted and collected in accordance with Example 2, step 2(v) (d)-(g) and surfactant recovery determined using an HPLC apparatus set up in accordance with Example 2, step 2 (vi) according to the protocol laid down in Example 2, steps (vii)-(ix).

(e) The recovery should be calculated from an extraction without the solvent wash under investigation.

Thus a skilled person is able to select an appropriate wash liquid, depending on the nature of the surfactant and the nature of the solid matrix being used. An appropriate wash may be sufficiently strong to precipitate the surfactant on the solid phase or sufficiently weak so as to minimise or prevent elution of the surfactant. Typically the wash liquid will be a water-insoluble organic solvent or water-soluble organic solvent.

A suitable wash liquid, particularly in the case of a surfactant having a poly (alkylene oxide) (such as poly (ethylene oxide)) group (e.g. polysorbate 80) may include hexane or the like, such as chloroform or toluene. A suitable wash liquid, particularly in the case of surfactant having a group that strongly binds the solid phase, such as a sorbitan group (e.g. polysorbate 80), can be a weak wash that does not elute the surfactant, such as acetonitrile, isopropanol and/or triethylamine. The skilled person will appreciate that, where appropriate, these approaches can be combined. For example, polysorbate 80 contains both a poly(ethylene oxide) group and a sorbitan group, and so both strong and weak washes can be used. For example, we have found that the following wash can be suitable for polysorbate 80: 30% (v/v) acetonitrile followed by isopropanol, 1% (v/v) triethylamine in hexane and finally hexane.

Further washing steps may be employed depending on the nature of the surfactant, the nature of the matrix and the nature of the proteinaceous components to be removed.

Following the washing step(s), the surfactant is typically eluted from the matrix and collected as an eluate. Any suitable eluent can be used. We have found a toluene:ethanol (1:1) mix provides good results in the exemplified system.

The purified liquid-phase surfactant preparation, or eluate derived therefrom, can be analysed in order to determine the surfactant content. The skilled person is well aware of methods to determine the surfactant content of a solution. For example, if a surfactant contains at least six alkylene oxide groups, then the surfactant can be complexed with ACT and surfactant concentration determined spectrophotometrically, for example as described by Garewal (*op. cit.*). Alternatively, surfactant content can be determined by HPLC, or aqueous GPC, such as described in the examples below.

Due to the low levels of proteinaceous component in the tested sample, the results of the analysis correlate more closely to the actual surfactant content of the initial liquid-phase surfactant preparation than if the analysis was performed according to methods of the prior art. Preferably, the level of proteinaceous component in the tested sample is below detectable levels when assessed by HPLC using the method exemplified below.

Accordingly, a method of the present invention can be useful wherein the liquid-phase surfactant preparation used is an aliquot of a larger preparation or one sample of a barch of preparations and the method comprises the additional step of correlating the thus determined surfactant content of the purified liquid-phase surfactant preparation, or eluate derived therefrom, with the surfactant content of the larger preparation or other batch members.

Having made this correlation, the user can then appropriately label the larger preparation or the other batch members, or can supply appropriate quality control reports, to reflect the thus determined surfactant content.

Since a method of the present invention provides a more accurate method for determining surfactant content than methods of the prior art, a preparation that has been subject to analysis using a method of the present invention and labelled with the thus determined surfactant content will be distinguished from prior art preparations in that its label or other associated data more accurately and more precisely reflects the surfactant level in its contents. Accordingly such a product is better able to comply with regulatory requirements.

A labelled liquid-phase surfactant preparation obtainable by a method as defined above is disclosed. It is preferred that, the liquid-phase surfactant preparation comprises a proteinaceous component, such as discussed above. Preferably the component is present in the liquid-phase surfactant preparation, of step (a) at a concentration of at least 50, 75, 100, 150, 200 mg/ml or more, where component levels are measured in weight per volume of surfactant preparation.

It may be appropriate to measure the ratio of surfactant to proteinaceous component in the liquid-phase surfactant preparation of step (a).

Accordingly, the ratio of surfactant to proteinaceous component, when expressed as mass of surfactant molecules per mass of proteinaceous component molecules (i.e. ppm) present in the liquid-phase surfactant preparation of step (a) may be less than 4,800 ppm, such as less than 4,500 ppm, 4,000 ppm, 3,500 ppm, 3,000 ppm, 2,500 ppm, 2,000 ppm, 1,500 ppm, 1,000 ppm, 900 ppm, 800 ppm, 700 ppm, 600 ppm, 500 ppm, 4.00 ppm, 300 ppm, 200 ppm, 110 ppm, 100 ppm, 90 ppm, 80 ppm, 75 ppm, 70 ppm, 60 ppm, 50 ppm, 40 ppm, 30 ppm, 20 ppm, 18 ppm, 17 ppm,

16 ppm, 15 ppm, 14 ppm, 13 ppm, 12 ppm, 11 ppm, 10 ppm, 9 ppm, 8 ppm, 7 ppm, 6 ppm ppm or less.

It will be apparent to the skilled reader that the methods described above are useful in the quality control of a batch of a surfactant-containing pharmaceutical preparation. Quality control is a system of maintaining standards in a manufactured product by testing a sample of the output of the process of manufacture, typically a lot or batch, against a standard specification, thereby ensuring the output product meets the required standards. This is particularly important in the manufacture of pharmaceutical products which need to match demanding regulatory requirements. Hence, the "component" in the context of the surfactant assay is generally the desired pharmaceutically active compound. Thus, quality control of the surfactant content of the preparation may be performed by determining the surfactant content of a sample of the preparation using a method as defined above.

Also diclosed is a surfactant-containing pharmaceutical preparation that has been quality-controlled using a method as described above.

The invention will now be described in more detail by reference to the following Figures and Examples wherein:

**Figure 1** shows the results of an assay of polysorbate 80 using metal ion complex formation and solvent extraction as described in Comparative Example 1.

**Figure 2** shows HPSEC chromatograms of different albumin extractions as described in Example 1.

**Figure 3** shows the HPLC set up used in Example 2.

**Figure 4A** shows the calibration data.

**Figure 4B** shows the linear calibration curve generated in Example 2.

**Figure 5** shows a chromatographic profile for calculating the theoretical plate number.

**Figure 6** shows a chromatographic profile for calculating peak tailing.

**Figure 7** shows a chromatographic profile for calculating resolution.

***Comparative Example 1***

The following example is based on the method of Garewal (*op. cit.*).

Samples of rHA preparation at 5 and 25% (w/v) were spiked with polysorbate 80 ("Tween 80" from Sigma) to a final concentration of 15μg.mL$^{-1}$ and 200μL aliquots were mixed with 2mL of ACT reagent (17.8g ammonium thiocyanate and 2.8g cobalt nitrate in 100mL Milli Q water).

The mixture was then extracted with 2mL of chloroform by mixing for 15 minutes at room temperature. The chloroform was then collected and the extraction repeated with a further four 1mL aliquots of chloroform.

The absorbance at 600nm of each chloroform extract was measured and the total absorbance for each sample calculated (i.e. the total ACT complex extracted under the defined conditions above).

This extraction procedure was repeated for standard polysorbate 80 solutions (0, 0.5, 5.0 and 50.0mg.mL$^{-1}$) prepared in both ultrapure water ("Milli Q™" water from Millipore Corp.) and rHA (5% w/v).

The results are shown in Table 1.

Table 1:

| Sample | Absorbance of Extract (600nm) | Tween 80 concentration from rHA standard curve (mg.mL$^{-1}$) | Tween 80 Recovery (%) |
|---|---|---|---|
| 5% (w/v) rHA + 15μg.mL$^{-1}$ polysorbate 80 | 0.329 | 0.610 | >4,000 |
| 25% (w/v) rHA + 15μg.mL$^{-1}$ polysorbate 80 | 0.346 | 0.695 | >4,600 |

ACT reagent when mixed with polysorbate 80 in aqueous solutions results in the formation of a coloured (blue) insoluble salt that can be extracted into organic buffers. The ACT reagent-solvent extraction of rHA produced higher

background absorbances than those obtained for water (Figure 1). This indicates that rHA contains substances other than polysorbate 80 that react with the ACT reagent producing a higher than expected absorbance in this method.

The estimation of polysorbate 80 in rHA final products is in excess of 4000 fold greater than the known concentration by this method (Table 1) demonstrating that the method is not accurate. The high absorbance in the unspiked rHA samples combined with the variability of the assay presumably accounts for this result. The high and variable interfering response obtained with unspiked rHA makes this method unsuitable for the direct assay of polysorbate 80 in rHA final products.

***Comparative Example 2***

In an attempt to overcome the contaminant problem experienced when using the method of Comparative Example 1, the effect of including an additional purification step, using a $C_{18}$ SPE cartridge, was assessed.

Polysorbate 80 was prepared in ultrapure water at a final concentration of 50mg.mL$^{-1}$ and in rHA at 15μg.mL$^{-1}$. To 200μL of each of these samples was added 800μL of ethanol followed by 2mL of ACT reagent. The mixtures were then extracted by the addition of 5mL of chloroform followed by mixing at room temperature for 15 minutes. The chloroform extracts were then removed and extracted on $C_{18}$ SPE as follows:

| Step | Procedure |
|---|---|
| *Wetting:* | 1 mL chloroform |
| *Equilibration:* | 1 mL chloroform |
| *Load:* | chloroform extract of ACT-Tween 80 complex from rHA or water |
| *Wash:* | 1 mL chloroform |
| *Elution:* | 0.25, 0.50 or 1.00mL of methanol |

SPE eluates were dried using centrifugal evaporation and resuspended in either 1mL (water extracts) or 0.5mL (rHA extracts) of tetrahydrofuran (THF). The absorbance at 600nm of each resuspended eluate was then measured.

As a control a 200μL aliquot of rHA containing no polysorbate 80 was also extracted using the procedure described above. The results are shown in Table 2.

*Table 2:*

| Sample | Elution Volume | $A_{600}$ | Polysorbate 80 recovery (%) |
|---|---|---|---|
| Water + 50mg.mL$^{-1}$ polysorbate 80 (Load control) | 1.0mL (volume dried down) | 1.463 | 100 |
| Water + 50mg.mL$^{-1}$ polysorbate 80 | 0.25mL | 1.297 | 89 |
| Water + 50mg.mL$^{-1}$ polysorbate 80 | 0.5mL | 1.246 | 85 |
| Water + 50mg.mL$^{-1}$ polysorbate 80 | 1.0mL | 1.170 | 80 |
| rHA (5% w/v) + 0μg.mL$^{-1}$ polysorbate 80 | 1.0mL | 1.132 | ND |
| rHA (5% w/v) + 15μg.mL$^{-1}$ polysorbate 80 | 1.0mL | 1.288 | ND |
| rHA (25% w/v) + 15μg.mL$^{-1}$ polysorbate 80 | 1.0mL | 1.144 | ND |

"ND" means not determined. The recoveries of these samples were not calculated as the absorption of the unspiked rHA is equivalent to the 50mg.mL$^{-1}$ standard. I.e. there is a high background from rHA samples.

Recovery of the ACT-polysorbate 80 complex from water is in excess of 80% (Table 2). This recovery of ACT-polysorbate 80 complex is maintained with SPE elution volumes from 0.25 to 1.0mL (Table 2). The use of low elution volumes may be beneficial in reducing the drying time of the eluates prior to resuspension.

Extraction of polysorbate 80 from rHA using ACT complex formation, solvent extraction and SPE resulted in essentially identical absorbances for samples with and without polysorbate 80 (Table 2). This indicates that the colour produced in these extracts is not totally related to the presence of polysorbate 80 and may be produced by rHA contaminants, excipients or the protein itself.

Thus, although polysorbate 80-ACT complex can be extracted from chloroform using $C_{18}$ SPE, a high background response is also produced.

***Comparative Example 3***

The method of assaying surfactant levels in solutions containing 'high' concentrations of protein (e.g. 52

$mg.mL^{-1}$) described by Lanteigne & Kobayashi (*op. cit.*) involves ethanol precipitation of protein, including overnight incubation of the sample at minus 30 °C (plus centrifugation and isolation of the supernatant), prior to complexing the surfactant by the addition of ACT and extraction of the ACT-surfactant complex using dichloromethane as an organic liquid phase.

We have found that the initial ethanol precipitation step proposed by Lanteigne & Kobayashi results in unacceptable levels of surfactant losses.

Aliquots (10mL) of rHA (5% w/v) + 10$\mu$g.$mL^{-1}$ polysorbate 80 were treated with 40mL of cold ethanol. Samples were centrifuged in a Sorval RC5C centrifuge (rotor = SS34) for 20 minutes at 20,000rpm. Supernatants were then dried using rotary evaporation (to approximately 2mL) and then extracted on $C_{18}$ SPE cartridges.

Removal of rHA by ethanol precipitation followed by $C_{18}$ SPE did not improve the polysorbate 80 recovery above 35% (data not shown). This indicates that the precipitation of protein results in losses of polysorbate 80 as the recovery on $C_{18}$ SPE is lower than that obtained from water extracts (i.e. with no protein).

The method of Garewal (*op. cit.*) also includes, as a first step, the addition of ethanol, albeit without the extensive overnight incubation of the sample at minus 30 °C as described by Lanteigne & Kobayashi. We have found that the initial ethanol addition step proposed by Garewal, or alternatively the addition of a similar solvent (in this case methanol or isopropanol), also results in unacceptable levels of surfactant losses.

rHA (5% w/v) + 10$\mu$g.$mL^{-1}$ polysorbate 80 was prepared and 10mL aliquots mixed with 5mL of either methanol, isopropanol or ethanol. The treated samples (15mL) were then extracted as above on $C_{18}$ SPE cartridges, eluates being assayed for polysorbate 80 as per Example 1. Pre-treatment of rHA final product with 30% isopropanol, methanol or ethanol prior to $C_{18}$ SPE resulted in recoveries of polysorbate 80 of 7, 25 and 51% respectively. These recoveries are unacceptably low for a regulatory assay and would lead to misleading results.

***Comparative Example 4***

In addition to the surfactant losses observed as a result of ethanol precipitation when using the method of Lanteigne & Kobayashi (*op. cit.*), described in Comparative Example 3, we have also demonstrated that the step of complexing the surfactant by the addition of ACT and extraction of the ACT-surfactant complex using dichloromethane as an organic liquid phase additionally causes surfactant loss.

Recovery of polysorbate 80 was compared between a 10mL aliquot of rHA (5% w/v) + 10$\mu$g.$mL^{-1}$ Tween 80 and a 10mL aliquot of ultrapure water + 10$\mu$g.$mL^{-1}$ polysorbate 80. Two additions of 70mL of ACT reagent (17.8g ammonium thiocyanate and 2.8g cobalt nitrate hexahydrate in 100mL ultrapure water) were made to each sample, prior to mixing with 52mL of dichloromethane. The samples were incubated overnight. Mixtures were centrifuged at 3000rpm for 5 minutes and the top aqueous phase discarded. A few crystals of anhydrous ammonium sulphate were added and the samples were mixed and re-centrifuged as above. The dichloromethane was then transferred to a clean tube and dried under a stream of helium. The residue was then resuspended in 1mL methanol and dried by centrifugal evaporation before being resuspended in 100mL THF. These resuspended samples were then assayed for polysorbate 80 as described in Example 1.

The recovery of polysorbate 80 from the water sample was 82%. The recovery of polysorbate 80 from the rHA sample was only 21 %. This demonstrates that the surfactant recovery protocol of Lanteigne & Kobayashi cannot efficiently extract surfactant in the presence of proteinaceous contaminant.

***Example 1***

Significant modifications to the methods used in Comparative Examples include -

- a protein-precipitating agent was added after, rather than before, the addition of ACT; and
- the ACT-polysorbate 80 complex was initially separated from proteinaceous components using centrifugation rather than solvent extraction.

Six lots ("A" to "F") of rHA were examined. Lot F was deliberately spiked with 15$\mu$g.$mL^{-1}$ polysorbate 80. To 10mL aliquots of the rHA (250mg.$mL^{-1}$), 2mL of ACT reagent followed by 18mL of acetone was added.

The samples were then vortex mixed and centrifuged at 47,800g for 15 minutes at 4°C. The supernatants were removed and diluted with 30mL of 100mM EDTA in 0.5M Tris/HCl buffer pH 8.0 (pre-extracted on a Bakerbond SDB 200mg/3mL column). These diluted samples were then extracted by solid phase extraction (SPE) using 50mg Bakerbond SDB1 columns as follows:

| Stage | Procedure |
|---|---|
| *Wetting:* | 2mL chloroform, 2mL methanol |
| *Equilibration:* | 2mL of 30% acetone, 50% EDTA solution, 20% ultrapure (Milli Q™) water |
| *Load:* | Diluted ACT supernatant |
| *Wash:* | 1mL each of ultrapure (Milli Q™) water, methanol, acetonitrile, iso-propanol, 0.5% ammonia in hexane, hexane, 1% acetic acid in hexane, hexane |
| *Elution:* | 2 × 750μL aliquots of toluene:ethanol (1:1) |

The SPE eluates were then dried by rotary evaporation, resuspended in 200μL of tetrahydrofuran (THF) and analysed by HPSEC, as follows:

| | |
|---|---|
| *Column:* | Three 300 × 7.8mm Phenomenex Phenogel 50Å, 5μm columns preceded by a 50 × 7.8mm 5μm guard column |
| *Mobile phase:* | tetrahydrofuran (THF) |
| *Flow rate:* | 1mL.min$^{-1}$ |
| *Injection:* | 50μL |
| *Detection:* | Waters 410 differential refractometer |
| *Column Temperature:* | 25°C |
| *Detector Temperature:* | 35°C |

Only slight contamination was observed in a couple of batches (E and D) (Figure 2). However, this can be negated in order to facilitate an accurate and precise assay by quantifying using height rather than area and/or using a standard curve in unformulated rHA (i.e. the standard curve can be prepared using the rHA prior to Polysorbate 80 being added).

***Example 2***

*1. Pharmaceutical preparations tested*

Orthoclone™ OKT3: The product literature for Orthoclone™ OKT3 ((muromonab-CD3)-Janssen-Cilag GmbH, Germany) states that each 5mL ampoule contains amongst other ingredients 1mg polysorbate 80. For analysis, 1.25mL of product was assayed, equivalent to 0.25mg polysorbate 80.

Vepesid™ J 100: ((etoposide)-Bristol Laboratories NJ, USA) contains amongst other ingredients 400mg polysorbate 80. For polysorbate 80 analysis, 5μL of product was assayed.

NovoSeven™ 240: ((Coagulation factor VIIa recombinant) Novo Nordisk A/S, Denmark) contains amongst other ingredients 0.65mg polysorbate 80. Reconstitution was performed as described in the product literature by the addition of 8.5mL of Sterile Water for Injection, USP. For polysorbate 80 analysis, 3mL of reconstituted product was assayed.

*2. Polysorbate 80 extraction and analysis*

Polysorbate 80 analysis was performed as follows:

(i) The following equipment was used: 1mL, 50mg Bakerbond SDB1 SPE cartridges (Mallinckrodt Baker B.V.); 3mL, 200mg Bakerbond SDB1 SPE cartridges (Mallinckrodt Baker B.V.); Analytical HPLC system with autoinjector fitted with 50μL sample loop, system controller and integrator; Refractive index detector suitable for HPLC system above; Phenomenex Phenogel 50Å, 5μm columns (300 x 7.8mm); Phenomenex Phenogel 50Å 5μm guard column (50 x 7.8mm); HPLC column heater and control module (Waters, without inserts); Sterilin containers (70mL); glass screw top vials (2mL - 12mm x 46mm) with lids; Univap rotary evaporative concentrator with rotor for 12mm x 46mm vials; 250μL glass HPLC sample vials with crimp top seals.

(ii) The following reagents were used: ammonium thiocyanate, AR grade (Fisher Chemicals); cobalt nitrate hexahydrate, AR grade (Fisher Chemicals); acetonitrile, far UV grade (Fisher Chemicals); ethylenediaminetetraacetic acid (disodium salt), Sigma Ultra grade (Sigma); tris(hydroxymethyl)aminomethane, Sigma grade (Sigma); hydrochloric acid (concentrated), SLR grade (Fisher Chemicals); hexane, Distol grade (Fisher Chemicals); tetrahydrofuran, GPC

grade (Fisher Chemicals); triethylamine, AR grade (Fisher Chemicals); isopropanol, HPLC grade (Fisher Chemicals); methanol, HPLC grade (Fisher Chemicals); chloroform, HPLC grade (Fisher Chemicals); water, laboratory grade; toluene, GPC grade (Fisher Chemicals); ethanol, AR grade (Fisher Chemicals); polysorbate 80, CAPP Raw material 34 (Surfachem); hexadecanoic acid, Sigma Ultra grade (Sigma).

(iii) The following solutions were used:

(a) Orthoclone™ OKT3, Vepesid™ J 100, and NovoSeven™ 240 as defined above, made up to 10mL with laboratory grade water;

(b) Aqueous solution of recombinant human albumin 25% (w/v) containing 15μg.mL$^{-1}$ Polysorbate 80 (10mL).

(c) ACT reagent (71.2g of ammonium thiocyanate and 11.2g of cobalt nitrate hexahydrate dissolved in 20mL of laboratory grade water, volume made up to 100mL).

(d) Buffered EDTA Solution (37.22g of ethylenediaminetetraacetic acid, disodium salt (EDTA), and 60.55g of tris(hydroxymethyl)aminomethane dissolved in approximately 900mL of laboratory grade water, pH adjusted to 8.0 by addition of concentrated hydrochloric acid and volume made up to 1L). The solution was purified using solid phase extraction as follows:

- A 3mL, 200mg Bakerbond SDB1 SPE cartridge was washed with 6mL of THF followed by 6mL of laboratory grade water allowing the solutions to flow through under gravity.
- A Pharmacia P1 pump was used to pass 6mL of the buffered EDTA solution through the SPE column at approximately 4 mL.min$^{-1}$ and the solution discarded.
- The remaining buffered EDTA solution was pumped through the SPE column at approximately 4 mL.min$^{-1}$ and collected for use in the assay.

(e) 30% (v/v) acetonitrile (30mL of acetonitrile was mixed with 70mL of laboratory grade water);

(f) 1% (v/v) triethylamine in hexane (200μL of triethylamine was dissolved in 19.8mL of hexane);

(g) toluene:ethanol (1:1) (10mL of toluene was mixed with 10mL of ethanol);

(h) polysorbate 80 standard solution (0.5000$\pm$0.0005g polysorbate 80 was dissolved in a final volume of 502mL of laboratory grade water in a grade A volumetric flask). Final concentration = 10mg.mL$^{-1}$; and

(i) "System Suitability" Standard Solution (0.10g hexadecanoic acid and 0.10g polysorbate 80 was dissolved in a final volume of 10mL of THF in a volumetric flask: Final concentration = 10mg.mL$^{-1}$ polysorbate 80 and 10mg.mL$^{-1}$ hexadecanoic acid; stored in 200μL aliquots in glass vials at minus 20°C).

(iv) Protein precipitation and removal was performed as follows:

(a) To all tubes (standards and tests) 4mL of ACT reagent was added and the mixture agitated gently to mix.

(b) To all tubes (standards and tests) 20mL of acetonitrile was added. Tubes were capped and shaken vigorously to break up viscous precipitates and vortex mixed for at least 1 minute. The tubes were incubated at room temperature for 15 minutes.

(c) Following incubation, each sample was vortex mixed for a further minute and then centrifuged at 47,800g (Sorvall RC5C Centrifuge and SS34 rotor at 20,000 r.p.m.) at 4°C for 20 minutes.

(d) To 10 Sterilin pots (70mL), 17mL of buffered EDTA solution was added.

(e) Following centrifugation, all of the supernatant from each tube was transferred into separate aliquots (17mL) of buffered EDTA solution (prepared above).

(f) Each centrifuge tube was rinsed with a further 172mL of buffered EDTA solution, which was added to the appropriate Sterilin pot. This represents the purified surfactant preparation.

(v) The purified surfactant preparation was further purified by Solid Phase Extraction (SPE). SPE was performed as follows:

(a) Following the manufacturer's instructions, ten 1mL, 50mg Bakerbond SDB 1 SPE cartridges were fitted to the SPE manifold and washed with 2mL chloroform followed by 2mL methanol and finally 2mL of 30% acetonitrile each.

(b) Each column was loaded (at approximately 0.5mL.min$^{-1}$) with a purified surfactant preparation obtained as outlined above.

(c) Each column was washed with 2mL of 30% (v/v) acetonitrile followed by 1mL isopropanol, 1mL of 1% (v/v) triethylamine in hexane and finally 1mL of hexane. The SPE cartridges were fully dried by passing air through each cartridge under vacuum for at least 30 seconds.

(d) Glass screw cap 2mL vials were fitted into the SPE manifold for eluate collection.

(e) Each column was eluted with 2 aliquots of 1000mL of toluene:ethanol (1:1) at approximately 0.5mL.min$^{-1}$. After each aliquot the eluate was expelled into the collection tube by passing a 10mL syringe full of air through the SPE column.

(f) All eluates were dried using centrifugal evaporation at 50°C under vacuum and then resuspended in 200 μL of THF.

(g) Each sample was transferred to 250μL glass HPLC vials and sealed with crimp top lids.

(vi) The HPLC apparatus was set up as shown in Figure 3. The mobile phase reservoir, containing 2L of tetrahydrofuran (THF), was placed in a thermostatically controlled water bath set to 25°C. A suction filter was connected to the HPLC Pump inlet pipe and the mobile phase primed the line up to the guard column placed to expel air. The autoinjector was connected to the guard column and then connected to the analytical columns. The guard and analytical columns were placed in the thermostatically controlled oven at 25°C as set on the Waters HPLC oven control module. One hour was allowed for temperature equilibration following installation of the columns. The outlet from the analytical column was connected to the refractive index detector inlet port, the refractive index detector reference was directed, and outlets purged into a waste container. The pump flow rate was set to 1.0 mL.min$^{-1}$ and the refractive index detector was set to a sensitivity of 256 and a time constant of 10 seconds. The detector oven was set to 35°C. The HPLC system controller and integrator were set to collect and integrate the chromatographic data following the manufacturer's instructions. Prior to analysis, system suitability tests were run (see below).

(vii) The following procedure was used for HPLC analysis: refractive index detector was purged for at least one hour prior to use and monitored for a steady baseline; buffered EDTA solution was prepared and its extraction started; all other assay buffers were prepared; extraction of tests and standards was started; the HPLC system was started and equilibrated; the baseline on HPLC was checked and test solution prepared; a system suitability test was run; while samples were being processed through SPE, the HPLC system was examined to ensure that the system suitability test was acceptable; when extraction was completed, and system suitability was acceptable, the samples were run.

(viii) An extracted standard curve of polysorbate 80 was prepared from 0.00, 0.10, 0.20, 0.30, 0.40 and 0.50mg polysorbate 80 in 10mL laboratory water.

(ix) Polysorbate 80 was quantified by HPLC as follows:

(a) Immediately after performing the system suitability test, 50μL of each sample was injected onto the HPLC under the standard conditions described above.

(b) After chromatography of all samples and the integration of the polysorbate 80 peaks, a linear calibration curve of polysorbate 80 peak height against standard concentration (mg.mL$^{-1}$) was constructed for the standards

by performing linear regression to calculate slope (m) and intercept on the x-axis (c) for the standard curve. These regression data were used to calculate the concentration of polysorbate 80 in the test samples as follows:

$$\text{Line of best fit for standard curve is polysorbate 80 peak height in test} = mx + c$$

Where x = polysorbate 80 concentration (μg.mL$^{-1}$)
Thus:

$$\text{Polysorbate 80 concentration } (\mu g.mL^{-1}) = \frac{(\text{Polysorbate 80 peak height in test} - c)}{m}$$

The mean polysorbate 80 concentration (μg.mL$^{-1}$) for the test replicates was then calculated.

*3. Results*

The extracted standard curve generated a linear calibration curve with a regression line $R^2$ of 0.999 and a percentage CV for the normalised peak heights of 3.4% (Figure 4). Comparison of the measured polysorbate 80 mass against the stated formulation mass using the calibration curve showed close agreement for Albumin, NovoSeven™ and Vepesid™ (Table 3).

*Table 3:*

| Product Trade name | Polysorbate 80 mass / vial (mg) | Polysorbate 80 mass / extract (mg) | Peak Height | Measure mass (mg) | Percentage of expected (%) |
|---|---|---|---|---|---|
| Albumin | 0.750 | 0.150 | 74975 | 0.15 | 100 |
| Orthoclone™ OKT3 | 1.0 | 0.25 | 102763 | 0.20 | 81 |
| Vepesid™ 100 | 400 | 0.40 | 202292 | 0.39 | 98 |
| NovoSeven™ 240 | 0.56 | 0.20 | 93038 | 0.18 | 90 |

The method of quantifying polysorbate 80 in rHA final product as described above showed itself to be suitable for the quantitation of all products with no modification to the methodology.

***System Suitability Tests***

*Procedure:*

1. 50μL of the System Suitability Standard Solution was injected onto the HPLC running under the standard conditions.

2. The test sample was evaluated by calculating the theoretical plates, tailing and resolution for polysorbate 80 and hexadecanoic acid (see below).

3. If any one of the parameters, either for the polysorbate 80 or hexadecanoic acid, was below the expected value the columns were replaced with a new set of columns.

*Evaluation Of Test Sample*

1. The theoretical plate number was calculated for both the polysorbate 80 (first eluting peak) and hexadecanoic acid (second eluting peak) peaks using Equation 1,with reference to Figure 5.

$$\text{Theoretical Plates} = 16 \times \left(\frac{t}{W_b}\right)^2 \qquad \textit{Equation 1}$$

Expected values: Polysorbate 80 > 650 theoretical plates
Hexadecanoic Acid > 6200 theoretical plates

2. The peak tailing was calculated for both the polysorbate 80 and hexadecanoic acid peaks using Equation 2, with reference to Figure 6.

$$\text{Tailing} = \frac{W_{0.05}}{2 \times f} \qquad \textit{Equation 2}$$

Expected values: Polysorbate 80 < 3.5
Hexadecanoic Acid < 3.0

3. The resolution between polysorbate 80 and hexadecanoic acid was calculated using Equation 3, with reference to Figure 7.

$$\text{Resolution} = 2 \times \frac{V_2 - V_1}{W_{b1} + W_{b2}} \qquad \textit{Equation 3}$$

Expected value: Resolution > 2.0

**Claims**

**1.** A method for removing a proteinaceous component from a liquid-phase surfactant preparation comprising -

(a) providing a liquid-phase surfactant preparation containing a proteinaceous component;
(b) adding a complexing agent to the preparation of step (a) and allowing the complexing agent to form a complex with the surfactant;
(c) simultaneously with step (b), or subsequently, adding a miscible precipitating agent to the preparation of step (a) or the product of step (b), respectively, to form a liquid-phase reaction mixture and allowing the miscible precipitating agent to precipitate the proteinaceous component within the liquid-phase reaction mixture; and
(d) separating the said complex from the precipitated proteinaceous component in the product of step (c) to provide a purified liquid-phase surfactant preparation;

wherein the complex remains in solution within the liquid-phase reaction mixture, and wherein step (d) retains the complex in the liquid phase.

**2.** A method according to Claim 1 wherein the surfactant contains one or more alkylene oxide groups.

**3.** A method according to Claim 1 or 2 wherein the surfactant is nonionic, and is preferably a condensate between an alkylphenol and an alkylene oxide; a polyoxyalkylene sorbitan oleate; or a polyoxyalkylene glycol.

**4.** A method according to Claim 2 or 3 wherein the complexing agent comprises a polyvalent metal ion, preferably a transition metal ion.

**5.** A method according to any preceding claim wherein the precipitating agent is an aqueous organic miscible solvent, such as an alcohol, cyanoalkyl, amine, amide, carboxylic acid, aldehyde, ketone, glycol, ether, alkylhalide or aromatic hydrocarbon, for example acetone, acetonitrile or ethanol.

**6.** A method according to any preceding claim wherein the proteinaceous component comprises a peptide, polypeptide or protein.

**7.** A method according to Claim 6 wherein the proteinaceous component comprises albumin, an albumin-containing fusion protein, a monoclonal antibody, etoposide or a blood clotting factor.

**8.** A method according to any preceding claim wherein the protein concentration of the proteinaceous component in the liquid-phase surfactant preparation of step (a) of Claim 1 is at least 50 mg/ml.

**9.** A method according to any preceding claim wherein the surfactant is present at less than 4800 ppm relative to the proteinaceous component in the liquid-phase surfactant preparation of step (a) of Claim 1.

**10.** A method according to any one of the preceding claims wherein the step of providing a purified liquid-phase surfactant preparation comprises centrifuging the reaction mixture, such that the precipitated proteinaceous component forms a pellet and the complex is retained in the supernatant, and separating the supernatant from the pellet.

**11.** A method according to any one of the preceding claims comprising the additional subsequent step of non-covalently binding the complex to a solid phase, preferably a solid phase extraction (SPE) medium.

**12.** A method according to Claim 11 wherein the complex is dissociated prior to the step of non-covalent binding of the surfactant to the solid phase.

**13.** A method according to Claim 12 wherein the complex is dissociated by the addition of a chelating agent to the purified liquid-phase surfactant.

**14.** A method according to any one of Claims 11 to 13 wherein the solid phase is a hydrophobic SPE medium.

**15.** A method according to Claim 14 wherein the SPE medium is a polystyrene divinylbenzene or a $C_{2-24}$alkyl medium.

**16.** A method according to any one of Claims 11 to 15 wherein the surfactant that is bound to the solid phase is washed to remove residual proteinaceous component.

**17.** A method according to Claim 16 wherein the matrix is washed with a water-soluble organic solvent, such as acetonitrile, isopropanol and/or triethylamine.

**18.** A method according to Claim 16 wherein the solid phase is washed with a liquid that would precipitate the surfactant if it were in solution.

**19.** A method according to Claim 18 wherein the solid phase is washed with a water-insoluble, organic solvent such as hexane, chloroform or toluene.

**20.** A method according to any one of Claims 11 to 19 wherein the surfactant is eluted from the solid phase and collected as an eluate.

**21.** A method according to any preceding claim comprising the additional step of determining the surfactant content of the purified liquid-phase surfactant preparation, or a further fraction derived therefrom.

**22.** A method according to Claim 21 wherein the liquid-phase surfactant preparation of step (a) is an aliquot of a larger

preparation or is one sample of a batch of preparations and the method comprises the additional step of correlating the thus determined surfactant content of the purified liquid-phase surfactant preparation, or a further fraction derived therefrom, with the surfactant content of the larger preparation or other batch members.

**23.** A method according to Claim 22 comprising the additional step of appropriately labelling the larger preparation or the other batch members to reflect the thus determined surfactant content.

**24.** A method of quality control of a batch of a surfactant-containing pharmaceutical preparation comprising determining the surfactant content of a sample of the preparation using a method according to any one of Claims 1 to 23.

**Patentansprüche**

**1.** Verfahren zum Entfernen eines Proteinbestandteils aus einer Flüssigphasentensidzubereitung, umfassend

(a) Bereitstellen einer Flüssigphasentensidzubereitung, enthaltend einen Proteinbestandteil;
(b) Hinzufügen eines Komplexbildners zu der Zubereitung von Schritt (a) und Erlauben des Komplexbildners einen Komplex mit dem Tensid zu bilden;
(c) gleichzeitig mit Schritt (b), oder anschließend daran, Hinzufügen eines mischbaren, präzipitierenden Mittels zu der Zubereitung von Schritt (a) bzw. dem Produkt von Schritt (b) um ein Flüssigphasenreaktionsgemisch zu bilden und Erlauben des mischbaren präzipitierenden Mittels, den Proteinbestandteil innerhalb des Flüssigphasenreaktionsgemisches zu präzipitieren; und
(d) Abtrennen des Komplexes von dem präzipitierten Proteinbestandteil in dem Produkt von Schritt (c), um eine gereinigte Flüssigphasentensidzubereitung bereitzustellen;

wobei der Komplex innerhalb des Flüssigphasenreaktionsgemisches in Lösung verbleibt und wobei Schritt (d) den Komplex in der flüssigen Phase zurückhält.

**2.** Verfahren nach Anspruch 1, wobei das Tensid eine oder mehrere Alkylenoxidgruppen enthält.

**3.** Verfahren nach Anspruch 1 oder 2, wobei das Tensid nichtionisch ist und bevorzugt ein Kondensat zwischen einem Alkylphenol und einem Alkylenoxid; einem Polyoxyalkylen-sorbitanoleat; oder einem Polyoxyalkylenglykol ist.

**4.** Verfahren nach Anspruch 2 oder 3, wobei der Komplexbildner ein polyvalentes Metallion, bevorzugt ein Übergangsmetallion umfasst.

**5.** Verfahren nach einem beliebigen vorhergehenden Anspruch, wobei das präzipitierende Mittel ein wasserhaltiges, organisches, mischbares Lösungsmittel ist, wie ein Alkohol, Cyanoalkyl, Amin, Amid, Carbonsäure, Aldehyd, Keton, Glykol, Ether, Alkylhalogenid oder aromatischer Kohlenwasserstoff, z. B. Aceton, Acetonitril oder Ethanol.

**6.** Verfahren nach einem beliebigen vorhergehenden Anspruch, wobei der Proteinbestandteil ein Peptid, Polypeptid oder Protein umfasst.

**7.** Verfahren nach Anspruch 6, wobei der Proteinbestandteil Albumin, ein Albumin enthaltendes Fusionsprotein, einen monoklonalen Antikörper, Etoposid oder einen Blutgerinnungsfaktor umfasst.

**8.** Verfahren nach einem beliebigen vorhergehenden Anspruch, wobei die Proteinkonzentration des Proteinbestandteils in der Flüssigphasentensidzubereitung von Schritt (a) von Anspruch 1 mindestens 50 mg/ml beträgt.

**9.** Verfahren nach einem beliebigen vorhergehenden Anspruch, wobei das Tensid in weniger als 4800 ppm in Bezug auf den Proteinbestandteil in der Flüssigphasentensidzubereitung von Schritt (a) des Anspruches 1 vorliegt.

**10.** Verfahren nach einem beliebigen der vorhergehenden Ansprüche, wobei der Schritt des Bereitstellens einer gereinigten Flüssigphasentensidzubereitung das Zentrifugieren des Reaktionsgemisches, so dass der präzipitierte Proteinbestandteil ein Pellet bildet und der Komplex in dem Überstand zurückgehalten wird, und das Abtrennen des Überstandes von dem Pellet umfasst.

**11.** Verfahren nach einem beliebigen der vorhergehenden Ansprüche, umfassend den zusätzlichen anschließenden

Schritt des nicht kovalenten Bindens des Komplexes an eine Festphase, bevorzugt ein Festphasenextraktions- (solid phase extraction, SPE) Medium.

**12.** Verfahren nach Anspruch 11, wobei der Komplex vor dem Schritt des nicht kovalenten Bindens des Tensids an die Festphase zur Dissoziation gebracht wird.

**13.** Verfahren nach Anspruch 12, wobei der Komplex durch die Hinzufügung eines Chelators zu dem gereinigten Flüssigphasentensid zur Dissoziation gebracht wird.

**14.** Verfahren nach einem beliebigen der Ansprüche 11 bis 13, wobei die Festphase ein hydrophobes SPE-Medium ist.

**15.** Verfahren nach Anspruch 14, wobei das SPE-Medium ein Polystyroldivinylbenzol- oder ein $C_{2-24}$-Alkylmedium ist.

**16.** Verfahren nach einem beliebigen der Ansprüche 11 bis 15, wobei das Tensid, das an die Festphase gebunden ist, gewaschen wird, um rückständigen Proteinbestandteil zu entfernen.

**17.** Verfahren nach Anspruch 16, wobei die Matrix mit einem wasserlöslichen organischen Lösungsmittel, wie Acetonitril, Isopropanol und/oder Triethylamin gewaschen wird.

**18.** Verfahren nach Anspruch 16, wobei die Festphase mit einer Flüssigkeit gewaschen wird, die das Tensid präzipitieren würde, wenn es in Lösung wäre.

**19.** Verfahren nach Anspruch 18, wobei die Festphase mit einem wasserunlöslichen organischen Lösungsmittel, wie Hexan, Chloroform oder Toluol gewaschen wird.

**20.** Verfahren nach einem beliebigen der Ansprüche 11 bis 19, wobei das Tensid von der Festphase eluiert wird und als ein Eluat gesammelt wird.

**21.** Verfahren nach einem beliebigen vorhergehenden Anspruch, umfassend den zusätzlichen Schritt des Bestimmens des Tensidgehaltes der gereinigten Flüssigphasentensidzubereitung oder einer weiteren davon abgeleiteten Fraktion.

**22.** Verfahren nach Anspruch 21, wobei die Flüssigphasentensidzubereitung von Schritt (a) ein Aliquot einer größeren Zubereitung ist oder eine Probe eines Batches von Zubereitungen ist und das Verfahren den zusätzlichen Schritt des Korrelierens des auf diese Weise bestimmten Tensidgehaltes der gereinigten Flüssigphasentensidzubereitung oder einer weiteren davon abgeleiteten Fraktion mit dem Tensidgehalt der größeren Zubereitung oder anderen Batchmitgliedern umfasst.

**23.** Verfahren nach Anspruch 22, umfassend den zusätzlichen Schritt des in geeigneter Weise Kennzeichnens der größeren Zubereitung oder der anderen Batchmitglieder, um den auf diese Weise bestimmten Tensidgehalt wiederzugeben.

**24.** Verfahren zur Qualitätskontrolle eines Batches einer Tensid enthaltenden pharmazeutischen Zubereitung, umfassend das Bestimmen des Tensidgehaltes einer Probe der Zubereitung unter Verwendung eines Verfahrens nach einem beliebigen der Ansprüche 1 bis 23.

## Revendications

**1.** Procédé d'élimination d'un constituant protéique d'une préparation de tensioactif en phase liquide comprenant les étapes consistant à :

(a) fournir une préparation de tensioactif en phase liquide contenant un constituant protéique ;
(b) ajouter un agent complexant à la préparation de l'étape (a) et permettre à l'agent complexant de former un complexe avec le tensioactif ;
(c) simultanément avec l'étape (b), ou subséquemment à celle-ci, ajouter un agent de précipitation miscible à la préparation de l'étape (a) ou au produit de l'étape (b), respectivement, pour former un mélange réactionnel en phase liquide, et permettre à l'agent de précipitation miscible de précipiter le constituant protéique dans le

mélange réactionnel en phase liquide; et

(d) séparer ledit complexe du constituant protéique précipite dans le produit de l'étape (c) pour fournir une préparation de tensioactif en phase liquide purifiée ;

dans lequel le complexe reste en solution dans le mélange réactionnel en phase liquide, et dans lequel l'étape (d) maintient le complexe en phase liquide.

**2.** Procédé selon la revendication 1, dans lequel le tensioactif contient un ou plusieurs groupe(s) d'oxyde d'alkylène.

**3.** Procédé selon la revendication 1 ou 2, dans lequel le tensioactif est non ionique, et est de préférence un condensé entre un alkylphénol et an oxyde d'alkylène ; un oléate de sorbitan de polyoxyalkylène ; ou un polyoxyalkylène glycol.

**4.** Procédé selon la revendication 2 ou 3, dans lequel l'agent complexant comprend un ion de métal polyvalent, de préférence un ion de métal de transition,

**5.** Procédé selon l'une des revendications précédentes, dans lequel l'agent de précipitation est un solvant organique et aqueux miscible, tel qu'un alcool, un cyanoalkyle, une amine, un amide, un acide carboxylique, un aldéhyde, une cétone, un glycol, un éther, un halogénure d'alkyl ou un hydrocarbure aromatique, par exemple l'acétone, l'acétonitrile ou l'éthanol.

**6.** Procédé selon des revendications précédentes, dans lequel le constituant protéique comprend un peptide, un polypeptide ou une protéine.

**7.** Procède selon la revendication 6, dans lequel le constituant protéique comprend de l'albumine, une protéine de fusion contenant de l'albumine, un anticorps monoclonal, un étoposide, ou un facteur de coagulation du sang.

**8.** Procède selon l'une des revendications précédentes, dans lequel la concentration en protéine du constituant protéique dans la préparation de tensioactif en phase liquide de l'étape (a) selon la revendication 1 est d'au moins 50 mg/ml.

**9.** Procède selon l'une des revendications précédentes, dans lequel le tensioactif est présent au moins à hauteur de 4 800 ppm par rapport au constituant protéique dans la préparation de tensioactif en phase liquide de l'étape (a) selon la revendication 1.

**10.** Procède selon l'une quelconque des revendications précédentes, dans lequel l'étape consistant à fournir une préparation de tensioactif en phase liquide purifiée comprend l'étape consistant à centrifuger le mélange réactionnel, de façon à ce que le constituant protéique précipite forme un culot et que le complexe soit maintenu dans le surnageant, puis à séparer le surnageant du culot.

**11.** Procédé selon l'une quelconque des revendications précédentes, comprenant l'étape suivante supplémentaire consistant à lier de façon non covalente le complexe à une phase solide, de préference à un milieu d'extraction en phase solide (SPE).

**12.** Procédé selon la revendication 11, dans lequel le complexe est dissocié avant l'étape consistant à lier de façon non covalente le tensioactif à la phase solide.

**13.** Procédé selon la revendication 12, dans lequel le complexe est dissocié en ajoutant un agent chélatant au tensioactif en phase liquide purifiée,

**14.** Procédé selon l'une quelconque des revendications 11 à 13, dans lequel la phase solide est un milieu SPE hydrophobe,

**15.** Procède selon la revendication 14, dans lequel le milieu SPE est un de polystyrène ou un milieu alkyle en $C_{2-24}$.

**16.** Procédé selon l'une quelconque des revendications 11 à 15, dans lequel le tensioactif qui est lié à la phase solide est lavé pour éliminer le constituant protéique résiduel.

**17.** Procédé selon la revendication 16, dans lequel la matrice est lavée avec un solvant organique hydrosoluble tel que

l'acétonitrile, l'isopropanol et/ou la triéthylamine.

**18.** Procédé selon la revendication 16, dans lequel la phase solide est lavée avec un liquide qui précipiterait le tensioactif s'il était en solution.

**19.** Procédé la revendication 18, dans lequel la phase solide est lavée avec un solvant organique insoluble dans l'eau, tel que l'hexane, le chloroforme ou le toluène.

**20.** Procédé selon l'une quelconque des revendications 11 à 19, dans lequel le tensioacif est élué à partir de la phase solide et collecté sous forme d'éluat.

**21.** Procède selon l'une quelconque des revendications précédentes comprenant l'étape supplémentaire consistant à déterminer la teneur en tensioactif de la préparation de tensioactif en phase liquide purifiée, ou d'une autre fraction dérivée de celle-ci.

**22.** Procédé selon la revendication 21, dans lequel la préparation de tensioactif en phase liquide de l'étape (a) est un aliquot d'une préparation plus grande ou est un échantillon d'un lot de préparations et le procédé comprend l'étape supplémentaire consistant à corréler la teneur en tensioactif de la préparation de tensioactif en phase liquide purifiée ainsi déterminée, ou d'une autre fraction dérivée de celle-ci, avec la teneur en tensioactif de la préparation plus grande ou des autres éléments du lot.

**23.** Procédé selon la revendication 22, comprenant l'étape supplémentaire consistant à marquer de façon appropriée la préparation plus grande ou les autres éléments du lot pour indiquer la teneur en tensioactif ainsi déterminée.

**24.** Procédé de contrôle de la qualité d'un lot de préparation pharmaceutique contenant un tensioactif, comprenant l'étape consistant à déterminer la teneur en tensioactif d'un échantillon de la préparation en utilisant un procédé selon l'une quelconque des revendications 1 à 23.

FIG. 1

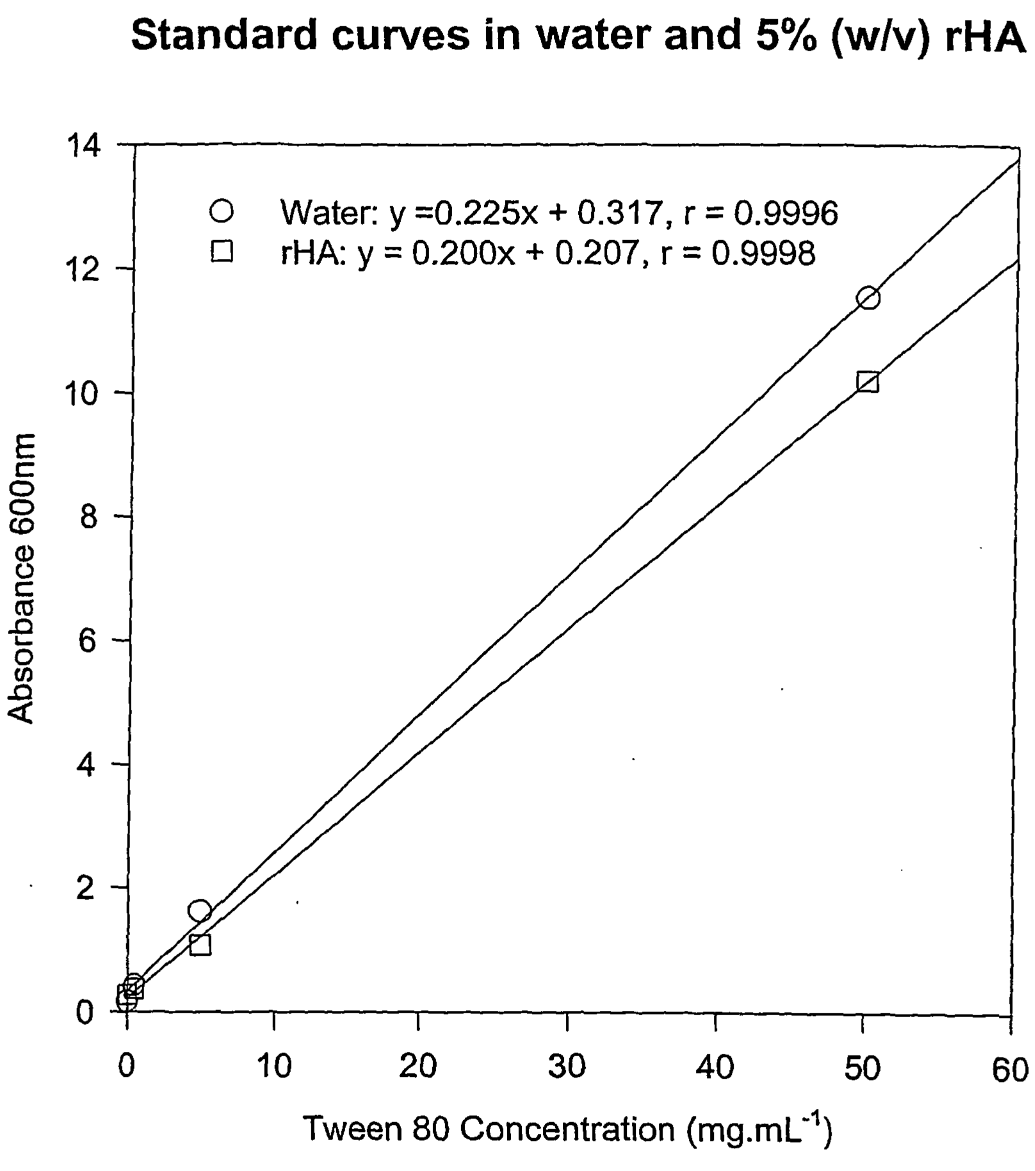
Standard curves in water and 5% (w/v) rHA
Water: y =0.225x + 0.317, r = 0.9996
rHA: y = 0.200x + 0.207, r = 0.9998
Absorbance 600nm
Tween 80 Concentration (mg.mL-1)
0
2
4
6
8
10
12
14
0
10
20
30
40
50
60

FIG.2

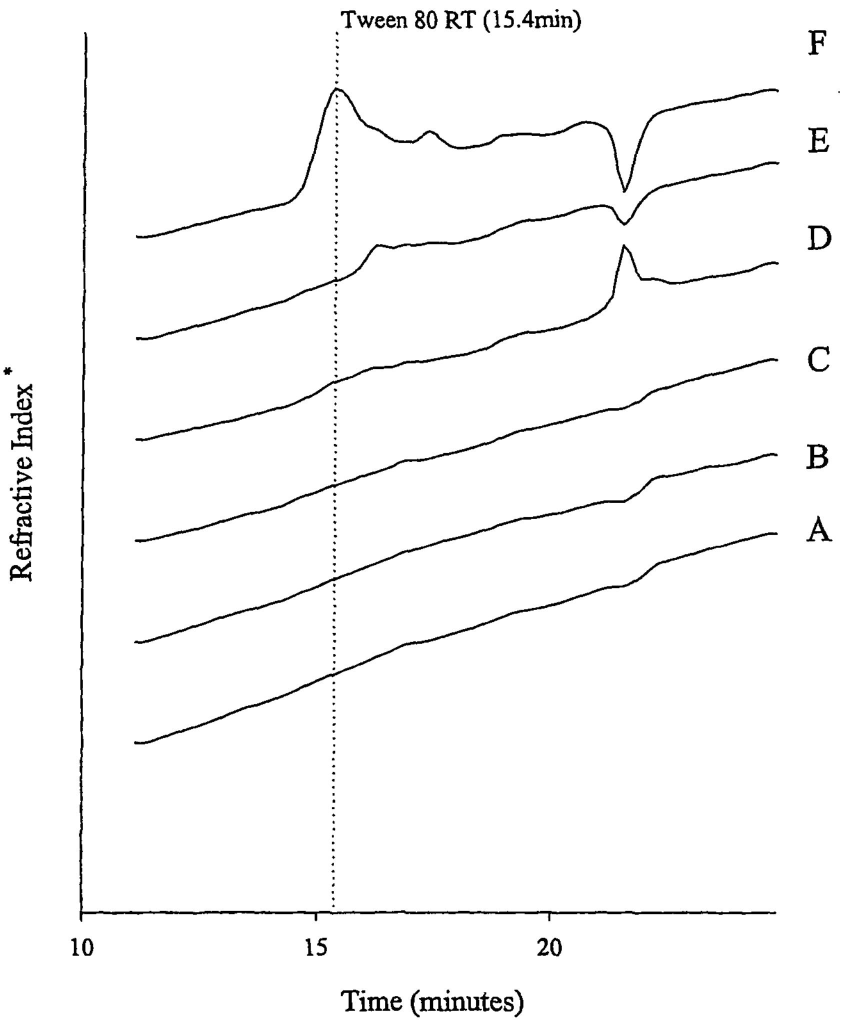
Tween 80 RT (15.4min)
F
E
D
C
B
A
Refractive Index *
10
15
20
Time (minutes)

FIG.3

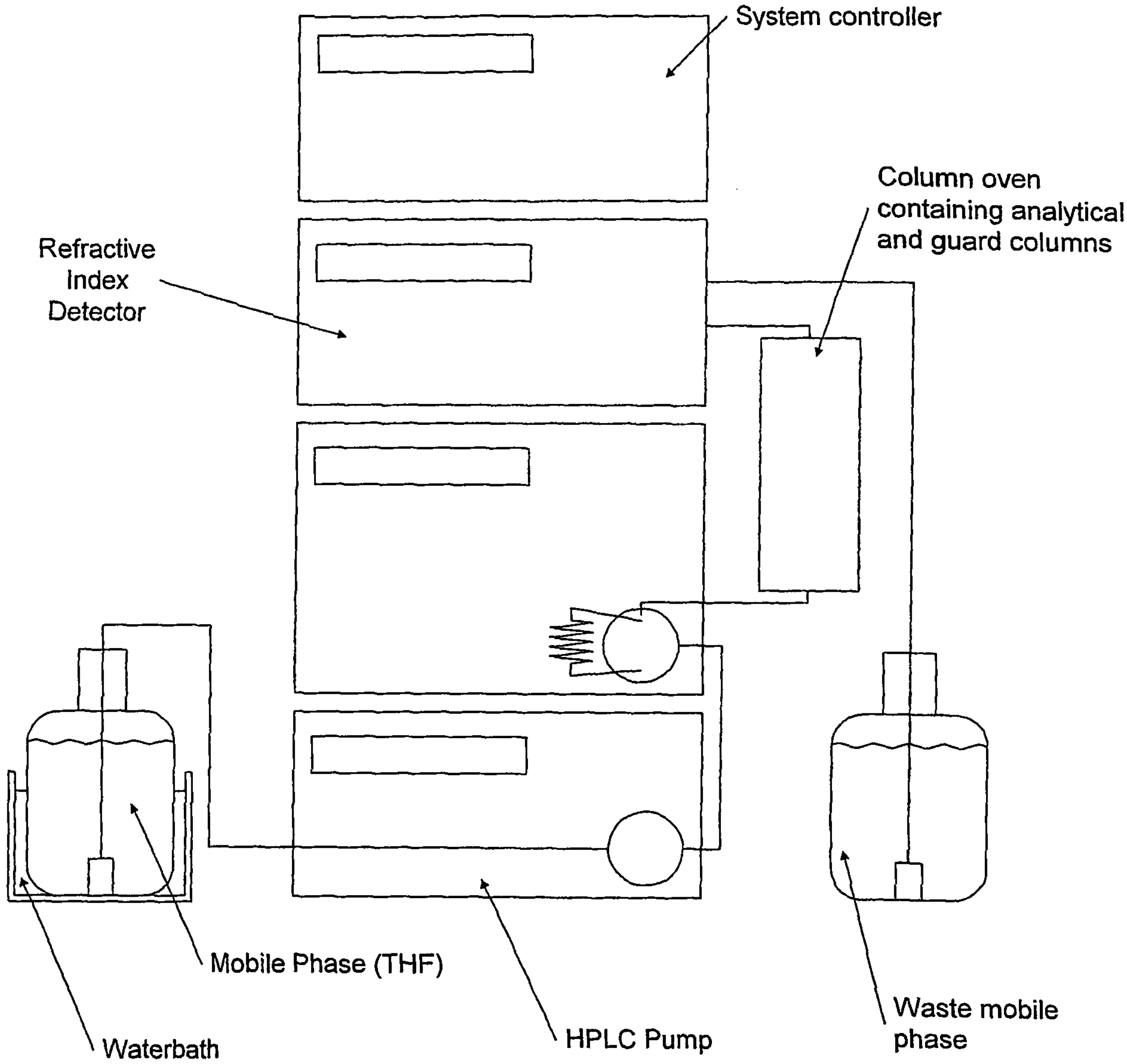
System controller
Column oven containing analytical and guard columns
Refractive Index Detector
Mobile Phase (THF)
Waterbath
HPLC Pump
Waste mobile phase

# FIG.4A

| Polysorbate 80 mass (mg) | Peak Height (μV) | Normalised Peak Height |
|---|---|---|
| 0 | 0 | N/A |
| 0.1 | 44956 | 485369 |
| 0.2 | 100007 | 517940 |
| 0.3 | 154746 | 527756 |
| 0.4 | 206210 | 524477 |
| 0.5 | 259541 | 526244 |
| | Percentage CV | 3.4 |

| Regression Data | |
|---|---|
| Slope = | 524630 |
| Intercept = | -3581 |
| $R^2$ = | 0.999 |

FIG.4B

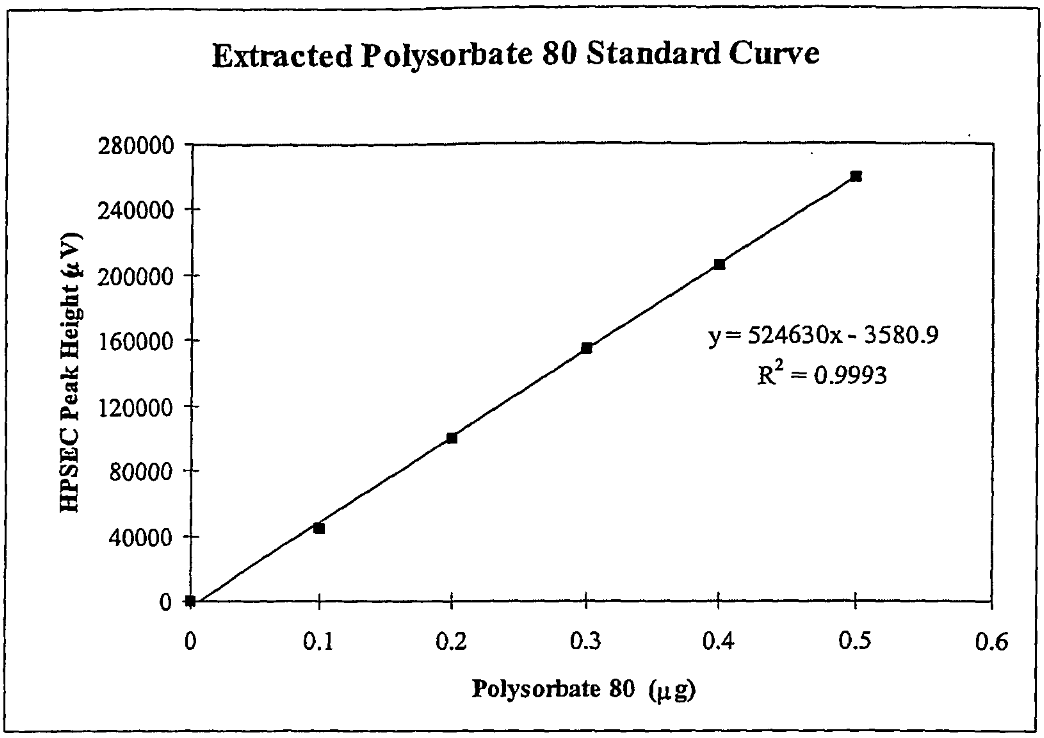
Extracted Polysorbate 80 Standard Curve
280000
240000
200000
160000
120000
80000
40000
0
HPSEC Peak Height (μV)
y = 524630x - 3580.9
R² = 0.9993
0
0.1
0.2
0.3
0.4
0.5
0.6
Polysorbate 80 (μg)

FIG.5

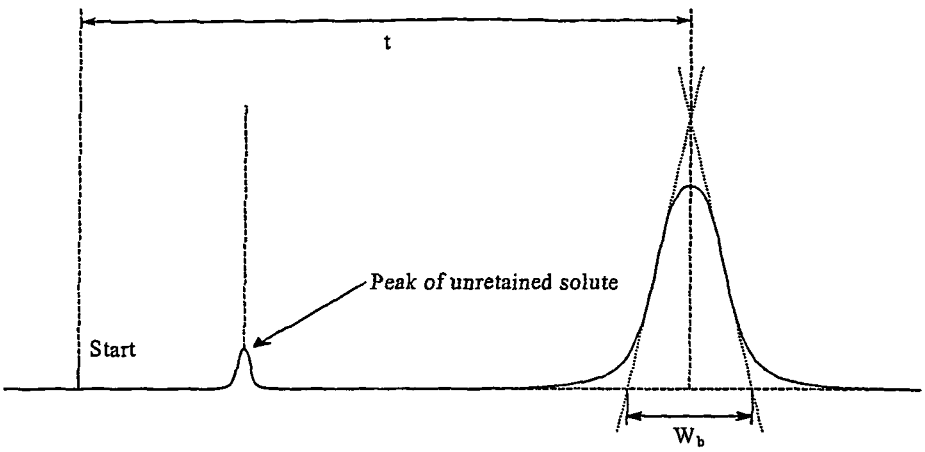

FIG.6

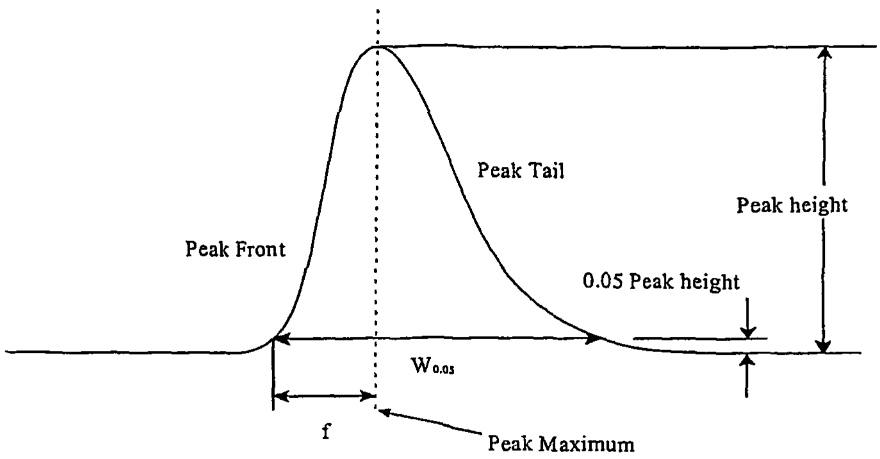
Peak Tail
Peak height
Peak Front
0.05 Peak height
W0.05
f
Peak Maximum

FIG.7

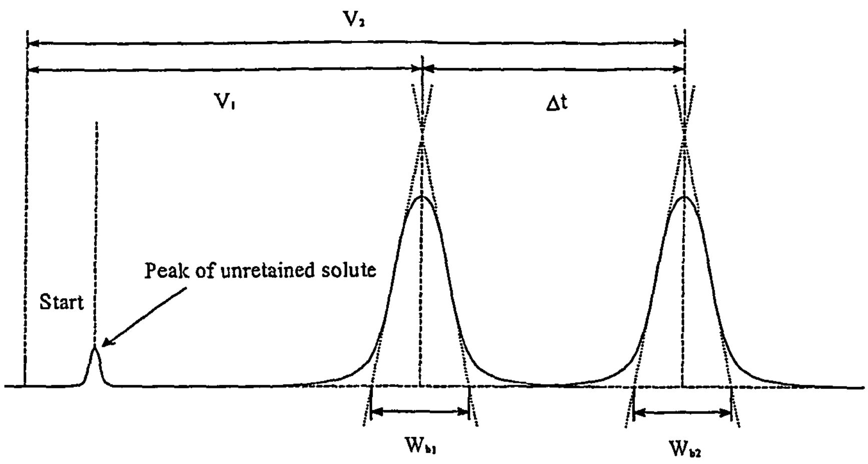

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0341103 A
- US 5389279 A
- US 5009814 A
- WO 0177137 A


### Non-patent literature cited in the description


- **MANNING, M.C. ; PATEL, K. ; BORCHART, R.T.** *Pharmaceutical Research,* 1989, vol. 6, 903-918
- **THUROW, H. ; GEISEN, K.** *Diabetologia,* 1984, vol. 27, 212-218
- **GAREWAL.** *Anal. Biochem.,* 1973, vol. 54, 319-324
- **LANTEIGNE, D. ; KOBAYASHI, K.** 6th Symposium on the Interface of Regulatory and Analytical Sciences for Biotechnology Health Products. Biogen, Inc, 27 January 2002
- Polyol Surfactants. **BECHER, P.** Nonionic Surfactants, Schick. Dekker, 1967, 247-299
- **CHISLETT, L.R. ; WALFORD, J.** *Int Flavours Food Addit.,* 1976, vol. 7, 61
- **VARMA, R.K. et al.** *Arzneimittel-Forsch,* 1985, vol. 35, 804
- **CRABB ; PERSINGER.** *Journal of the American Oil Chemist's Society,* 1961, vol. 41, 752-755